# EUROPEAN PATENT APPLICATION

(11) **EP 4 342 451 A1**
(43) Date of publication of application: **27.03.2024**
(21) Application number: 22382874.0
(22) Date of filing: 22.09.2022
(51) Int. Cl.: A61K 9/00, A61K 31/00, A61K 9/08

(54) **PHARMACEUTICAL COMPOSITIONS AND MANUFACTURING METHODS THEREOF**

(71) Applicant: Galenicum Health SLU, 08950 Esplugues de Llobregat (ES)
(72) Inventor: Gómez Coello, Luis, San Agustín del Guadalix (ES); Pérez Pérez, María del Rocío, San Agustín del Guadalix (ES)
(74) Representative: Galenicum Health S.L.U.

(57) **Abstract**

The present invention relates to a pharmaceutical composition comprising methylene blue. Manufacturing processes of such compositions are also described.

## Description

### FIELD

The present invention relates to a pharmaceutical composition comprising methylene blue. Manufacturing processes of such compositions are also described.

### STATE OF THE ART

Methylthioninium chloride, also known as methylene blue or, 3,7-bis- (dimethylamino)-phenothiazin-5-ium chloride, is an oxidation-reduction agent used for the treatment of patients with acquired methemoglobinemia.

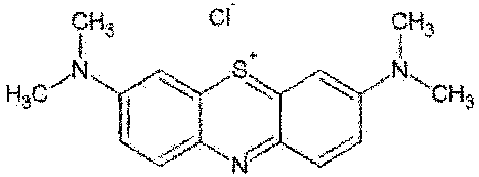

Methylene blue is an old known compound. According to Merck Index, 8th Edition (1964), it was first described in a German Patent in 1877 (Badische Anilin und Soda Fabirk, 1877).

Methylene Blue compounds used in the present invention can be obtained according to the processes disclosed in WO 2018/167185.

WO 2006/032879 and WO 2008/007074 disclose a high purity diaminophenothiazonium compound with a purity greater than 98%, an Azure B impurity content of less than 2% and a low content of certain metals. Moreover, this document discloses that in the different steps of the process, care is taken to use non-metallic materials, reagents and solvents free of metal residues and that the purification and hydration steps are performed in an enamelled reactor under nitrogen.

WO 2008/006979 discloses a method for preparing methylene blue with a low level of impurities and low metal content.

The European Public Assessment report for Methylthioninium chloride Proveblue discloses that methylene blue has a very strong affinity for metals and that it is difficult to manufacture a substance that complies with the requirements of Ph. Eur. Moreover, it discloses that methylene blue even absorbs metals from the brown vials where it is stored.

There is a need in the field of pharmaceutical manufacturing for a stable, safe and effective pharmaceutical composition of methylene blue wherein said methylene blue contains a higher level of impurities than the prior art.

### SUMMARY OF THE INVENTION

The present invention discloses pharmaceutical compositions comprising methylene blue and methods for preparing said compositions, wherein the active substance has significantly higher impurity levels and metal content than those identified in the prior art. The inventors have surprisingly found that a very specific pH is required in said pharmaceutical compositions in order to obtain a stable pharmaceutical composition.

The first aspect of the invention deals with a sterile aqueous pharmaceutical composition comprising methylene blue, wherein said methylene blue has an Azure B impurity content ranging between 2% and 3% and wherein the pH of said composition is below 4.0.

A second aspect of the invention deals with a process for the preparation of said pharmaceutical composition.

### DESCRIPTION

### Definitions

As used in the present disclosure, the following words, phrases, and symbols are generally intended to have the meanings as set forth below, except to the extent that the context in which they are used indicates otherwise.

All percentages are expressed by weight (w/w) unless specifically noted otherwise.

The term "active ingredient" as used herein refers to a therapeutically active compound, as well as any pharmaceutically acceptable hydrates and solvates of the compound.

The term "pH adjuster" as used herein refers to pharmaceutically acceptable excipients which are added to the solution of the active agent to adjust the pH to a certain value. Such pH adjusters can be alkaline or acid agents and may comprise inorganic salts as well as organic acids or salts of organic acids. Additionally, the pH adjusters may be present in the form of a buffer.

The term "pharmaceutically acceptable" as used herein indicates that the substance or composition must be compatible chemically and/or toxicologically, with the other ingredients comprising a formulation, and/or the mammal being treated therewith.

The term "effective" amount as used herein refers to an amount of a compound, agent or substance that is of sufficient quantity to result in a quantifiable effect, e.g., a shift in the pH.

The term "solution" as used herein refers to a liquid preparation of one or more soluble chemical substances, which are dissolved in water.

The term "suitable for parenteral administration" as used herein refers to a sterile solution that can be administered parenterally to a patient. Sterility is defined as the absence of living organisms. The conditions of the sterility test are given in the European Pharmacopoeia 10th edition.

The term "suitable pharmaceutical container" as used herein refers to a container for pharmaceutical use is an article which holds or is intended to contain and protect a drug and is or may be in direct contact with it. The container and its closure must not interact physically or chemically with the substance within in any way that would alter its quality.

The term "vial" as used herein refers to a vial suitable for containing injectable pharmaceutical compositions according to the European Pharmacopoeia 9th edition, Chapter 3.2.1 "Glass Containers for Pharmaceutical Use".

The term "terminal sterilization" as used herein refers to a process whereby a product is sterilized in its final container or packaging, which permits the measurement and evaluation of quantifiable microbial lethality. The probability of viable microorganisms being present on a product unit after exposure to the proper sterilization process should be less than 10⁻⁶.

The term "bioburden" as used herein refers to the population of viable microorganisms on or in raw materials, products, or labeling/packaging materials determined before sterilization. Bioburden analysis is carried out according to US Pharmacopoeia <71> Sterility tests.

The term "blanketing" as used herein refers to the act of maintaining an inert atmosphere of nitrogen gas (N₂) during storage and processing.

The term "0.2 µm pore filter", as used herein refer to filter where the nominal pore rating have been determined to be 0.2 µm or less, according to ISO 13408-2:2018 Aseptic processing of health care products.

Unless otherwise indicated, all the analysis methods are carried out according to the European Pharmacopoeia 10th edition and USP monograph for Methylene Blue Injection (USPNF 2022 Issue 1).

The terms "Azure B impurity" or "Azure B" as used herein refer to 3-(dimethylamino)-7-(methylamino)phenothiazin-5-ylium. Percentage of Azure B is determined by HPLC (Column XBridge Phenyl 100, flow rate 1.0 mL/min, UV detector wavelength of 246 nm, column temperature 30ºC, mobile phase: 0.1% trifluoroacetic acid in water:acetonitrile (75:25% v/v), run time 15 minutes).

The term "Azure B impurity content" refers to the amount of Azure B with respect to the active ingredient (w/w).

The terms "purity" or "chemical purity" referred to herein refers to the measure of an element containing a single substance without any other element tarnishing its standalone existence. In the present invention, the purity calculations have been provided based on the total organic impurity content and the metal content.

The terms "metal purity" or "metal content" referred to herein pertains to the amounts of the eleven (11) metals specified by the European Pharmacopoeia: Al, Cr, Zn, Cu, Fe, Mn, Ni, Mo, Cd, Sn, and Pb. The metal content is expressed relative to the amount of the active ingredient. The metal content has been determined following Ph. Eur. 10th Edition, chapter 2.4.20 - Determination of elemental impurities.

The inventors have found out that a sterile aqueous pharmaceutical composition comprising methylene blue as active ingredient wherein the active ingredient used to manufacture has not a high level of purity as the methylene blue obtained as per the prior art can be surprisingly stable when the pH of said composition is below 4.0.

In a first aspect of the invention, a sterile aqueous pharmaceutical composition comprising methylene blue as active ingredient wherein the Azure B impurity content of said composition ranges from 2.0% to 3.0% and the pH of said composition is below 4.0.

In an embodiment of the first aspect of the invention, said aqueous pharmaceutical composition comprises an Azure B impurity content ranging from 2.1% to 3.0%, preferably from 2.3% to 3.0%.

In another embodiment, the Azure B impurity content can be taken as representative of the total impurity content of the pharmaceutical composition.

In another embodiment, the pH of said pharmaceutical composition ranges from 3.2 to 3.9, preferably from 3.3 to 3.8.

In another embodiment, the said pharmaceutical composition has a concentration of 5 mg/mL of methylene blue or any of its hydrates.

In another embodiment, the metal content relative to the amount of methylene blue is above 20 ppm, preferably above 50 ppm, more preferably above 100 ppm.

In another embodiment, the iron (Fe) content relative to the amount of methylene blue is above 20 ppm, preferably above 50 ppm, more preferably above 100 ppm.

In another embodiment, said pharmaceutical composition is packaged in a glass vial sealed with a rubber stopper, or in a pre-filled-syringe, or in a cartridge or in a glass ampule, preferably the composition is packaged in a glass vial sealed with a rubber stopper.

In another embodiment, said pharmaceutical composition is to be parenterally administered to a human.

In another embodiment, said pharmaceutical composition is to be used as a medicament.

In another embodiment, said pharmaceutical composition is to be used in the treatment of dementia, cognitive impairment, Alzheimer or methemoglobinemia, preferably for the treatment of methemoglobinemia.

In another embodiment, the compositions show assay values within 95%-105% and total organic impurity content below 3% after 6 months storage under 40ºC/75% RH packaged in glass vials with rubber stopper.

All the embodiments of the first aspect of the invention may be combined between them.

In a second aspect of the invention, a process for preparing said pharmaceutical composition is disclosed.

In an embodiment of the second aspect of the invention, a process for preparing the pharmaceutical composition of the first aspect of the invention comprising the steps of:
a. weighing the methylene blue,
b. in a reactor, dissolving and mixing methylene blue of step a. in water to obtain a solution
c. optionally, adjusting the pH of the solution
d. optionally, filtering the obtained solution,
e. filling the solution into a suitable pharmaceutical container,
f. sterilising the obtained solution

In a further embodiment, the methylene blue in step a. has a purity below 98% and an Azure B content of at least 2%.

In a further embodiment, the reactor of step b. is a reactor made of metal, preferably stainless steel.

In a further embodiment, the pH of step c. is optionally adjusted to a range between 3.2 and 3.9. Preferred pH adjusters are hydrochloric acid 0.1 N and sodium hydroxide 0.1 N.

In a further embodiment, the filtration of step d. is filtered through a 0.2 µm pore size filter.

In a further embodiment, the filtration of step d. is performed twice.

In a further embodiment, the bioburden of the solution before any filtration is higher than 10 CFU/ 100 mL. In case the bioburden before filtration is not more than 10 CFU/ 100 mL. the filtration stages may be waived.

In a further embodiment, step f. is performed by terminal sterilisation and preferably reaching at least 110ºC for not less than 10 minutes, more preferably reaching at least 120ºC for not less than 10 minutes.

In a further embodiment, the terminal sterilisation of step f. is performed in an autoclave.

In a further embodiment, the aqueous solution reaches a temperature inside the reactor of at least 20ºC.

In a further embodiment, the suitable pharmaceutical container of step e. is a glass vial sealed with a rubber stopper.

In a further embodiment, the vial is blanketed with nitrogen before sealing.

In a further embodiment, the sterilisation process reaches at least 121ºC for at least 10 minutes, preferably 20 minutes.

In a further embodiment the sterilisation process is maintained between 121ºC and 124ºC in an autoclave during 20 minutes.

The embodiments of the second aspect of the invention may be combined between them to obtain the composition according to the first aspect of the invention. A preferred combination of embodiments is as follows:

A process for preparing a sterile aqueous pharmaceutical composition comprising methylene blue as active ingredient wherein the Azure B impurity content of said composition ranges from 2.0% to 3.0% and the pH of said composition is below 4.0 comprising the steps of:
a. weighing the methylene blue having a purity below 98% and an Azure B content of at least 2%,
b. dissolving and mixing in water methylene blue in a stainless steel reactor to obtain a solution,
c. optionally, adjusting the pH of the solution to a range between 3.2 and 3.9,
d. optionally, filtering the obtained solution,
e. filling the solution into a suitable pharmaceutical container,
f. sterilising the obtained solution, preferably by terminal sterilisation and preferably reaching at least 110ºC for not less than 10 minutes, more preferably reaching at least 120ºC for not less than 10 minutes.

In another possible combination of the embodiments of the second aspect of the invention, the process is as follows:
1. Procedure of weighing and sampling methylene blue
   1. Transfer the active ingredient methylene blue to a controlled area and weigh the active ingredient to reach a final concentration of 5 mg/mL (weight 5 mg of methylene blue or any of its hydrates for each mL of solution to be manufactured). Protect the active ingredient from light during the whole manufacturing process.
2. Preparation of the solution
   1. Add water for injection (approximately 88% of the batch size volume)
   2. Add slowly the total amount of methylene blue sodium into the vessel under continuous stirring until the total dissolution of the API.
   3. Once the active ingredient is completely dissolved, set (if necessary) the temperature of the solution at 22 ± 3ºC.
   4. Check the pH of the solution and adjust, if necessary, the pH of the solution with hydrochloric acid 0.1 N and/or sodium hydroxide 0.1 N to pH target below 4.0.
   5. Make up to the final volume with water for injection
   6. Stir the solution for not less than 20 minutes in order to ensure complete homogenization of the solution.
3. Filtration Stage I
   1. Transfer the solution from the stainless steel compounding vessel to a stainless steel holding vessel through a 0.2 µm pore size filter.
4. Filtration Stage II
   1. Transfer the solution from the stainless steel holding vessel to a stainless steel filling vessel through a 0.2 µm pore size filter.
5. Filling of the vials
   1. Fill aseptically 1 mL, 2 mL or 10 mL of the active solution into 10 mL type I clear glass vials and close them with a stopper and an aluminium seal.
6. Terminal sterilisation
   1. Place the filled vials in the autoclave
   2. Sterilise for 20 minutes between 121ºC and 124ºC.
   3. Cool down the sterilised vials to room temperature.
   4. Perform visual inspection of the vials and store them protected from light.

### EXAMPLES

### I. Preparation of Methylene Blue Injection USP compositions at 5 mg/mL

Methylene blue pharmaceutical compositions 1 to 5 for parenteral administration were prepared. General preparation method is described below:
- Weight the necessary amount of methylene blue to reach a final concentration of 5 mg/ml
- Add water for injection in a stainless steel vessel
- Add slowly the total amount of methylene blue into the vessel under continuous stirring until the total dissolution of the methylene blue
- Once the active ingredient is completely dissolved, check the pH of the solution and adjust, if necessary.
- Add water for injection until to reach the final volume
- Stir the resulting solution to ensure complete homogenization of the solution
- Filter twice the solution through a 0.2 µm pore size filter
- Fill vials with the obtained solution and seal them with a rubber stopper
- Place the filled vials in the autoclave and sterilise them

### II. Analysis of the prepared compositions

Compositions 1 to 5 were prepared as described in previous point I. Analise what and when

| | Comp 1 | Comp 2 | Comp 3 | Comp 4 | Comp 5 |
|---|---|---|---|---|---|
| Reactor material | Stainless steel | Glass | Stainless steel | Stainless steel | Stainless steel |
| pH | 4.1 | 4.6 | 4.6 | 3.2 | 3.9 |
| Azure B impurity (%) | 1.9 | 2.4 | 2.5 | 2.3 | 2.4 |
| Total impurity content (%) | 1.9 | 2.4 | 2.5 | 2.3 | 2.4 |
| Metal content (ppm) | 23.63 | 131.0 | 105.2 | 192.8 | 131.2 |
| Assay (%) | 94.7 | 96.6 | 95.6 | 100.1 | 100.1 |

### III. Stability of the prepared compositions

During manufacturing of all the compositions, we did not identify a significant increase in the total organic impurities before and after manufacturing. That is, the organic impurities carried by the active ingredient did not increase during manufacturing.

## Claims

1. A sterile aqueous pharmaceutical composition comprising methylene blue as active ingredient, wherein
a. the Azure B impurity content of said composition ranges from 2.0% to 3.0% and,
b. the pH of said composition is below 4.0

2. The pharmaceutical composition according to the preceding claim wherein the Azure B impurity content of said composition ranges from 2.1% to 3.0%, preferably from 2.3% to 3.0%.

3. The pharmaceutical composition according to any of the preceding claims wherein the pH is from 3.2 to 3.9, preferably from 3.3 to 3.8.

4. The pharmaceutical composition according to any of the preceding claims wherein the metal content in the composition is above 20 ppm, preferably above 50 ppm, more preferably above 100 ppm.

5. The pharmaceutical composition according to the preceding claim wherein the Iron (Fe) content with respect to the active ingredient is above 20 ppm, preferably above 50 ppm, more preferably above 100 ppm.

6. The pharmaceutical composition according to any of the preceding claims wherein the pharmaceutical composition is packaged in a glass vial, or in a pre-filled-syringe, or in a cartridge or in an ampule, preferably the composition is packaged in a glass vial and sealed with a rubber stopper.

7. The pharmaceutical composition according to any of the previous claims wherein the composition is to be parenterally administered to a human.

8. The pharmaceutical composition according to any of the preceding claims for use as a medicament.

9. The pharmaceutical composition according to any of the preceding claims for the use in the treatment of dementia, cognitive impairment, Alzheimer or methemoglobinemia.

10. A process for preparing a pharmaceutical composition according to any of the preceding claims comprising the steps of:
a. weighing the methylene blue,
b. in a reactor, dissolving and mixing methylene blue of step a. in water to obtain a solution
c. optionally, adjusting the pH of the solution
d. optionally, filtering the obtained solution,
e. filling the solution into a suitable pharmaceutical container,
f. sterilising the obtained solution

11. A process according to the preceding claim wherein the reactor material is metallic, preferably stainless steel.

12. A process according to any of the two preceding claims wherein the solution of step c) is filtered through a 0.2 µm pore size filter.

13. The process according to the preceding claim, wherein the filtration is performed twice.

14. A process according to any of the **four** preceding claims, wherein the suitable pharmaceutical container is a glass vial sealed with a rubber stopper.

15. A process according to any of the **five** preceding claims, wherein the pH of the aqueous solution ranges between 3.2 and 3.9.
